# EUROPÄISCHE PATENTSCHRIFT

(11) **EP 1 331 934 B1**
(45) Veröffentlichungstag und Bekanntmachung des Hinweises auf die Patenterteilung: **29.03.2006**
(21) Anmeldenummer: 00987112.0
(22) Anmeldetag: 09.11.2000
(51) Int. Cl.: A61K 31/215, A61K 31/505, A61P 17/00

(54) **PPAR-ALPHA,BETA-AKTIVATOREN ZUR BEHANDLUNG VON ALOPECIA AREATA UND VITILIGO**
PPAR-ALPHA,BETA ACTIVATORS FOR TREATING ALOPECIA AREATA AND VITILIGO
ACTIVATEURS DE PPAR-ALPHA,BETA DESTINES AU TRAITEMENT DE L'ALOPECIE AREATA ET DU VITILIGO

(43) Veröffentlichungstag der Anmeldung: 06.08.2003
(73) Patentinhaber: Phenion GmbH & Co KG, 60439 Frankfurt (DE)
(72) Erfinder: KIPPENBERGER, Stefan, 60316 Frankfurt am Main (DE); LOITSCH, Stefan, M., 60329 Frankfurt am Main (DE); BERND, August, 55411 Bingen-Kempten (DE)
(74) Vertreter: Kucken, Stefan
(86) Internationale Anmeldenummer: PCT/DE2000/003955
(87) Internationale Veröffentlichungsnummer: WO 2002/038150

(56) Entgegenhaltungen:
- WO-A-00/49992
- WO-A-00/62766
- WO-A-98/32444
- WO-A-99/34783
- DE-A- 10 053 003
- US-A- 6 060 515

## Beschreibung

Die vorliegende Erfindung betrifft die Verwendung von Aktivatoren von Peroxisom-Proliferator-aktivierten Rezeptoren α und β (auch als δ bekannt) zur Herstellung einer Zubereitung zur, Behandlung immunologisch bedingter Hautstörungen und zur Herstellung von Arzneimitteln, die zur Behandlung immunologisch bedingter Hautstörungen geeignet sind, bei der die Hautstörungen durch eine der Erkrankungen aus der gruppe hervorgerufen werden, die aus Alopecia aerata und Vitiligo besteht..

Bisherige Behandlungsmethoden von immunologisch bedingten Hautstörungen setzten Kortikosteroide oder Cyclosporin A ein, um die Immunreaktion des Körpers zu unterdrücken. Nachteilig an der Verwendung von Cyclosporin A ist, daß es nicht topisch anwendbar ist, sondern systemisch verarbreicht werden muß. Ebenso unerwünscht sind Nebenwirkungen der Kortikosteroide wie Kortison, z.B. die Atrophie der Haut.

WO98/57631 offenbart die Anwendung von Tetrazolidindion, einem PFAR-Agonisten zur Unterdrückung der entzündlichen Cytokinproduktion, die mit dem Immundeffiziensyndrom, Multipler Sklerose, rheumatoider Arthritis in Verbindung steht. Bevorzugt wird insbesondere die orale Verarbreiehung.

WO98/32444 offenbart die Anwendung einer Vielzahl von PPARα-Aktivatoren zur Behandlung infektiöser und ekzematöser Hauterkrankungen sowie Krebserkrankungen der Haut und bullösen Erkrankungen. Der experimentelle Nachweis der Wirkung der eingesetzten PPAR-Aktivatoren zeigt die Verminderung des trans-epidermalen Wasserverlustes berauspräparierter Hautstückchen von Rattenfeten. PPAR-Aktivatoren führen zu einer Steigerung der Barrierefunktion der Haut.

WO97/25042 offenbart die Verwendung von PPARα- und δ-Aktivatoren zur Behandlung von Hyperglykämie, Typ-II-Diabetes und Diabetesfolgeerkrankungen und insbesondere die Behandlung von oder Prophylaxe des X-Syndroms. Weiterhin werden spezifische Verbindungen offenbart, die als PPARα- und δ-Aktivatoren wirken.

Jiang, C. et al., Nature (1998). 391, 82-86 beschreiben die Unterdrückung der Cytokinsynthese in isolierten Monozyten durch PPARγ-Aktivatoren, wenn die Zellen mit Pherbolmyristylacetat induziert werden. PPARα-Aktivatoren, z.B. WY 14643 (Pirinixinsäure), oder l.eukotrien B₄ zeigten keine Aktivität zur Unterdrückung der Cytokinproduktion.

Die WO99/34783 offenbart die Verwendung des PPARγ-Aktivators zur Behandlung von Hautanomalien, die mit der Differenzierung von Epidermiszellen in Verbindung stehen. Für bestimmic PPARγ-Aktivatoren wird der Einfluß auf die Differenzierung von normalen menschlichen Keratinozyten nachgewiesen und daraus die Eignung dieser Substanzen zur Behandlung von entzündlichen Hautkrankheiten, beispielsweise auch nach Sonnenbestrahlung, hergeleitet.

Die EP 0 749 752 A1 offenbart allgemein die synergistische Wirkung der medizinischen Anwendung eines Liganden der Steroid-/ Thyroidrezeptoren bei der Heterodimerisierung von speezifischen Rezeptoren mit dem RXR-Rezeptor, jeweils mit gebundenem Liganden. Beispielhaft genannt werden Liganden für den Retinsäure-Rezeptor, den Vitamin D-Rezeptor, den Trijodthyroxin-Rezeptor und den (PPAR)-Rezeptor im Zusammenspiel mit jeweils Liganden für den RXR-Rezeptor. Die therapeutische Anwendung wird in der Behandlung verschiedener Anomalien und Krankheiten der Haut, Hautalterungserscheinungen sowie der Alopecie, Cellulitis, Obesitas, und aktinischer Keratosen gesehen. Als spezifischer Ligand für den PPAR-Rezeptor wird die Brompalmitinsaure angeführt.

Die DE 36 21 861 A1 offenbart die Verwendung verschiedener lipidsenkender Mittel zur Behandlung dermatologischen Erkrankungen, insbesondere Clofibrat zur Verwendung bei Alopecia mucinosa.

Die Aufgabe, die sich vorliegend stellt, ist das Bereitstellen geeigneler Wirkstoffe, die zur Behandlung immunologisch bedingter Hauterkrankungen topisch angewandt werden können und vorzugsweise äußerlich aufzutragen sind. Weiterhin ist es wünschenswert, Nebenwirkungen bisheriger immunsupprimierender Substanzen zu verringern bzw. zu vermeiden.

Die: erfindungsgemäße Lösung liegt in der Anwendung von Aktivatoren der Peroxisom-Proliferator-aktivierten Rezeptoren (PPAR) α und β (auch δ genannt) zur Herstellung einer Zubereitung zur Behandlung von immunnlogisch bedingten Hautstörungen bzw. zur Herstellung von Arzneimitteln mit einem Gehalt an PPARα- und β-Aktivatoren. Im Sinne dieser Erfindung können die Aktivatoren für PPAR α und β sowohl einzeln, insbesondere PPARβ-Aktivatoren, besonders bevorzugt PPARα-Aktivatoren einzeln oder als Gruppe von PPARα-Aktivatoren, als auch in Kombination miteinander, also PPARα- und β-Aktivatoren zusammen, eingesetzt werden.

Erfindungsgemäß zu verwendende Aktivatoren von Peroxisom-Proliferator-Aktivierten Rezeptoren (PPARα,β-Aktivatoren)sind insbesondere Pirinixinsäure (WY 14.643), Clofibral, Leukotrien B4, 8-S-Hydroxycicosatetraenylsäure (8-S-HETE) und Prostaglandin A2. Erfindungsgemäße PPARrα,β-Aktivatoren sind überdies Substanzen, die direkt oder erst nach metabolischer Umsetzung als Liganden für PPARα,β dienen können, oder die solche Substanzen in der Zelle induzieren, die dann als Ligenden für PPARα-β wirken können.

Erfindungsgemäße Arzneimittel zur topischen Anwendung umfassen in ihrer Formulierung üblichc Trägerstoffe, Haltbarkeitsmittel, Emulgatoren, Parfümstoffe und gegebenenfalls weitere bekannte Zusatzstoffe, Bei äußerlich anzuwendenden Präparaten zur Verwendung bei UV-Bestrahlung, z. B. Sonnenschutzmitteln, können weiterhin vor UV-Stahlung schützende Substanzen enthalten sein.

Autoimmunkrankheiter der Haut, die erfindungsgemäß behandelbar sind, sind Alopecia areata und Vitiligo.

Bei krankheitsbedingten, autoimmunologischen Reaktionen der Haut führt die topische Anwendung des PPARα-Aktivators Pirinixinsäure zur Minderung der immunologisch bedingten Symptome des Anschwellens und der Rötung der Haut.

Wie im Folgenden für die PPARα,β-Aktivatoren Pirinixinsäure bzw. Clofibrat nachgewiesen wird, liegt der erfindungsgemäßen Anwendung der PPARα,β-Aktivatoren deren gemeinsamer Wirkmechanismus auf die Synthese von Cytokinen, insbesondere auf die Transkription der Gene, die für Interleukin-6 und Interleukin-8 codieren, zugrunde. So kann durch die Anwendung von PPARα,β-Aktivatoren die Induktion der lnterleukine-6 und -8 gehemmt werden und damit die Wirkung dieser Cytokine unterdrückt werden. Die pleintrophen Wirkungen von 1L-6 auf die Polyfraktionen und Differenzierung von B-Zellen und seine Rolle bei der Entwicklung des Schmerzempfindens bzw. der Entwicklung von Überempfindlichkeiten gegen äußere Reize, cie zu Schmerz führt, sowie die pleiotrophe Wirkung von IL-8 unter anderem als Chemoattraktant. für T-Zellen und Neutrophile sind die Grundlage der Anwendung von PPARα,β-Aktivatoren bei den erfindungsgemäßen verschiedenen Indikationsgebieten, da bei jeder dieser lndikationen eine Beteiligung von IL-6 und IL-8 auftritt, Entsprechend läßt sich durch die Steuerung d. h. die Verminderung der Induktion dieser beiden Cytokine, deren Einfluß bei entzündlichen Hautreaktionen, die Abstoßung von Transplantaten der Haut, Alterserscheinungen, die zumindest teilweise auf Belastungen durch Licht zurückzuführen sind sowie bei Neuropathien, insbesondere Schmerz, regulieren.

### Figurenbeschreibung

- Figur 1: ist eine graphische Darstellung der durch UVB-Bestrahlung bei 150 mJ/cm² für 165 Sekunden hervorgerufenen Konzentration von Interleukin 6 gemessen im Abstand von 24 Stunder nach der Bestrahlung unter dem Einfluß von Pirinixinsäure bzw. Clofibral.
- Figur 2: ist eine graphische Darstellung der durch UVB-Bestrahlung bei 150 mJ/cm² fur 165 Sekunden hervorgerufenen Konzentration von lnterleukin 8, gemessen im Abstand von 24 Stunden nach der Bestrahlung unter dem Einfluß von Pirinixinsäure bzw. Clofibrat.
- Figur 3: ist eine graphische Darstellung des Anteiles geschädigter Zellen, die durch steigende Konzentrationen an Pirinixinsäure bzw. Clofibrat geschädigt werden.

Die folgenden Beispiele erläutern die Erfindung im größeren Detail:

### Beispiel 1: UV-Bestrahlung von menschlichen Keratinozyten

Menschliche Keratinozyten der Zellinie HaCaT wurden zu 2 x 10⁶ Zellen/9,6 cm² in Kulturgefäßen ausgesät und für 24 Stunden inkubiert. 4 Stunden vor der Bestrahlung wurde das Kulturmedium durch frisches Kulturmedium, das mit 200 µM Pirinixinsäure supplementiert war, ersetzt Für die Bestrahlung mit UVB bei 150 mJ/cm² wurde das Medium durch Phosphatpuffer ersetzt, der ebenfalls mit 200 µM Pirinixinsäure supplementiert war. Nach einer Bestrahlung für 165 Sekunden wird der Phosphatpuffer gegen Kulturmedium mit 200 µM Pirinixinsäure ausgetauscht. Im Anschlaß an 6 Stunden lnkubation werden die Zellen lysiert und die Gesamt-RNA isoliert. Die RNA wurde in cDNA umgeschrieben und die Transkripte für IL-6 und IL-8 durch kompetive Multiplex PCR bestimmt (Kippenberger et al., J. Invest.Dermatol.1998; 110 (4), 364-367). Die semiquantitative Bestimmung erfolgte im Verhältnis zum konstitutiv exprimierten Gen der Glyzerinaldehyddehydrogenase. Dieses Modell wird herangezogen, um die entzündungshemmende Wirkung von Arzneimitteln zu testen (Stein. M. et al., Arzneimittel-Forschung/Drug Kes. 47, 1266-1270).

Die Verminderung der Konzentrationen von Interleukin 6 und Interleukin 8 anhand der jeweiligen mRNA, die durch UVB-Bestrahlung induziert werden, ist in der folgenden Tabelle dargestellt:

| | ohne Bestrahlung | | Bestrahlung mit UVB | |
|---|---|---|---|---|
| | Ohne Supplementierung | supplementiert mit 200 µM Pirinixinsäure | ohne Supplementierung | Supplementiert Mit 200 µM Pirinixinsäure |
| Interleukin 6 | 100 % | 115% | 1979 % | 325% |
| Interleukin 8 | 100% 49% | | 180 % | 45% |

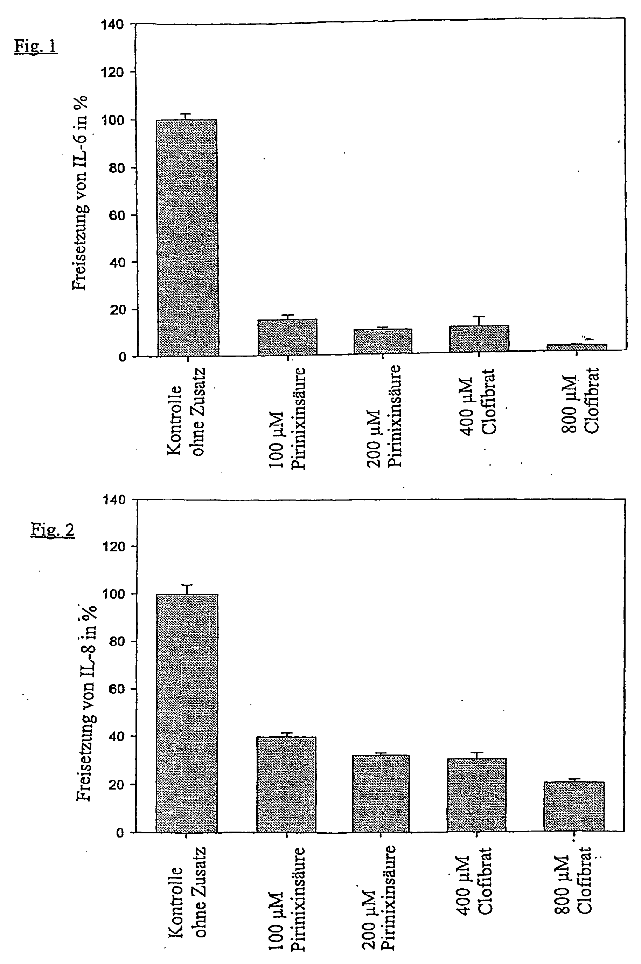

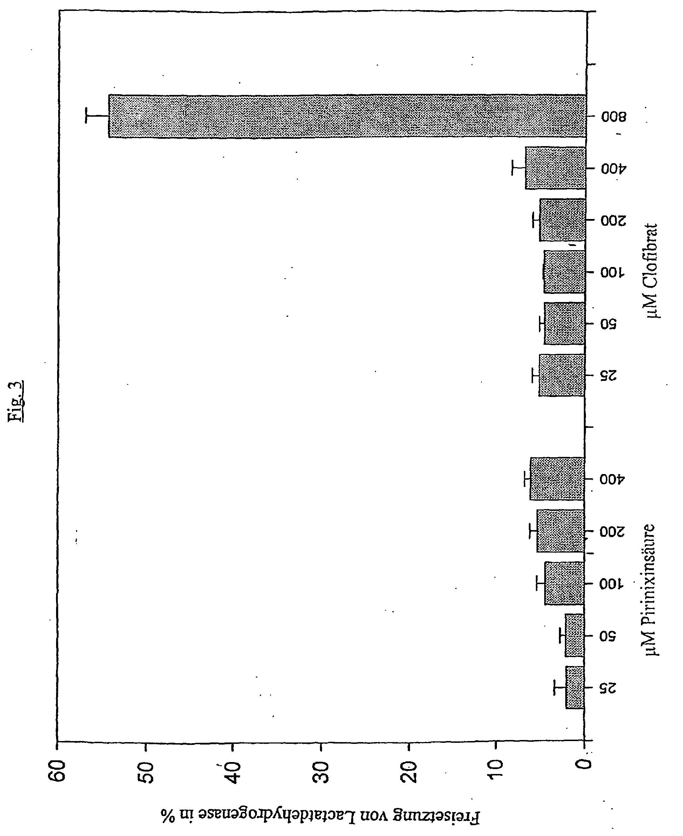

Die direkte Konzentrationsbestimmung der durch die UVB-Bestrahlung induzierten Konzentration der Interleukine 6 und 8, graphisch dargestellt in Figuren 1 und 2, belegt die Hemmung der Produktion beider Interleukine durch Pirinixinsäure bzw. Clofibrat.

Für diesen Nachweis wurden IIaCaT-Zellen wie oben beschrieben bestrahlt und 24 h danach der Überstand zur Messung freigesetzter Interleukine IL-6 und IL-8 eingesetzt. IL-6 und IL-3 wurden per ELISA (Fa. Cell Concept, Umkirch, Deutschland, bzw. Fa. Promocell, Heidelberg. Deutschland) bestimmt.

Ohne Zusatz von PPAR-Aktivatoren zeigen die Zellen ohne Bestrahlung extrazelluläre Interlenkin 6-Konzentrationen von etwa 36 pg/ml. Werden die unbestrahlten Zellen den PPARα,β-Aktivatoren Pirinixinsäure oder Clofibrat ausgesetzt, so haben diese nur einen geringen Einfluß auf die Sekretion von Interleukin 6. Die Bestrahlung der Zellen in Abwesenheit von PPAR-Aktivatoren mit UVB bewirkt eine Induktion von Interleukin 6 auf etwa 2700 pg/ml. Der Zusatz von Pirinixinsäure oder Clofibrat unterdrückt die durch die UVB-Bestrahlung induzierte Sekretion von Interleukin 6 auf unter 15 %.

Unbestrahlte Zellen produzieren eine extrazelluläre Konzentration an Interleukin 8 von etwa 1800 pg/ml. Die Zugabe von Pirinixinsäure auf 100 µM (200 µM) bremst die Sekretion von Interleukin 8 geringfügig. Die Zugabe von Clofibrat hingegen reduziert die extrazelluläre Interleukin 8-Konzentration auf unter 20 % bei unbestrahlten Zellen. Die Bestrahlung mit UVB (150 mJ/cm²) induziert eine extrazelluläre Konzentration von Interleukin 8 von etwa 3700 pg/ml. Die zugesetzten PPAR-Aktivatoren Pirinixinsäure bzw. Clofibral unterdrücken die Induktion der Sekretion von Interleukin 8 auf einen Wert von unter 40 %.

Bereits das *in vitro* Modell mit menschlichen Keratinozyten zeigt, daß der PPARα-Aktivator Pirinixinsäure geeignet ist, die immunologischen Reaktionen, die durch schädliche UVB-Bestrahlung hervorgerufen werden, deutlich zu reduzieren. Die beispielhaft gemessener, Konzentrationen der Interleukine 6 und 8 belegen die zentrale Wirkung von PPARα- und β-Aktivatoren auf den Ablauf der immunologischen Antwort, die sich auf äußere Reize einstellt. Da Interleukin 8 allgemein als Chemoattraktant für T-Zellen und Neutrophile sowie die Wirkung von Interleukin 6 auf die Proliferation und Differenzierung von B-Zellen auch bei Autoimmunerkrankungen der Haut eine zentrale Rolle spielen, lassen sich PPARα- und β-Aktivatoren zur Behandlung dieser Erkrankungen der Haut einsetzen.

### Beispiel 2: Einfluß vor Pirinixinsäure auf die Hautrötung durch UVB-Bestrahlung.

Als Beispiel für die Wirksamkeit der erfindungsgemäßen PPARα,β-Aktivatoren zur Unterdrükkung von Entzündungsreaktionen wurde der Einfluß oberflächlich aufgetragener Pirinixinsäure in Salbengrundlage mit anschließender Bestrahlung mit UVB getestet. Pirinixinsäure wurde zweiprozentig in Dorithin® Salbengrundlage (Astra Medica. Wien) eingebracht und anschließend auf die Haut der Unterarminnenseite dünn aufgetragen und für vier Stunden abgedeckt gelassen. Die so vorbereitete Haut wurde einer Bestrahlung mit UVB bei 90, 76,5. 63, 49,5 und 36 mJ/cm² ausgesetzt. Zum vergleich wurde unbehandelte Haut in gleicher Weise bestrahlt. Nach 18 Stunden nach der Bestrahlung konnte bei beiden Versuchspersonen eine Hautrötung erst bei einer Bestrahlung von mindestens 63 mJ/cm² bei der mit Pirinixisäure-Salbe behandelten Haut festgestellt werden, wohingegen unbehandelte Haut bereits bei 36 mJ/cm² UVB Erytheme bildete.

### Beispiel 3: Zellschädigung durch Pirinixinsäure und Clofibrat

HaCaT-Zellen wurden für 24 Stunden mit 25, 50, 100, 200 und 400 µM Pirinixinsäure bzw. 25, 50, 100, 200, 400 und 800 µM Clolibrat inkubiert und anschließend wurden zellfreic Überstände entnommen. Die indirekte Bestimmung der Laktatdehydrogenase erfolgte über den chromogenen Nachweis von NADH₂⁺, das aus zugesetztem NAD⁺ gebildet wurde, und dient als Maß für die Membrangeschädigung der Zellen. Die Darstellung zeigt die Laktatdehydrogenase-Freisetzung als Anteil der Laktatdehydrogenase-Konzentration der gesamten Zellen nach deren anschließender Lyse. Wie aus Figur 3 ersichtlich, sind Konzentrationen von bis zu 400 µM Pirinixinsäure bxw. Clofibrat verbältnismäßig unschädlich für kultivierte Zellen.

## Patentansprüche

1. Verwendung von PPARα,β-Aktivatoren zur Herstellung einer Zubereitung zur Behandlung immunologisch bedingter Hautstörungen, bei der die Hautstörungen durch eine der Erkrankungen aus der Gruppe hervorgerufen werden, die aus Alopecia areata und Vitiligo besteht.

2. Verwendung nach Anspruch 1, wobei die Zubereitung in einer Form zur topischen Anwendung ist.

3. Verwendung von PPARα,β-Aktivatoren nach einem der vorhergehenden Ansprüche, bei der die PPARα,β-Aktivatoren aus der Gruppe ausgewählt werden, die aus Pirinixinsäure, Clofibrat, Leukotrien B4, 8-S-Hydroxyeicosatetraenylsäure, Bezafibrat, Ciglitazon und Prostaglandin A2 oder deren pharmakologisch annehmbaren Salzen und deren Mischungen bestehen.

## Claims

1. Use of PPAR-α,β-activators for the production of a preparation for treating immunologically caused skin disorders, in which the skin disorders are caused by one of the diseases from the group consisting of Alopecia areata and Vitiligo.

2. Use claimed in claim 1, in which the preparation is in a form for topical application.

3. Use of PPAR-α,β-activators as claimed in any of the preceding claims, in which the PPAR-α,β-activators are selected from the group consisting of pinnixic acid, clofibrate, leucotriene B4, 8-S-hydroxyeicosatetraenylic acid, bezafibrate, ciglitazone and prostaglandin A2 or pharmaceutically acceptable salts thereof and mixtures of these PPAR-α,β-activators.

## Revendications

1. Utilisation d'activateurs de PPAR-α,β pour l'obtention d'une préparation destinée au traitement d'affections dermiques dues à des troubles du système immunologique, ces affections dermiques appartenant au groupe constitué par l'alopecia areata et le vitiligo.

2. Utilisation selon la revendication 1, dans laquelle la préparation est sous une forme destinée à l'application locale.

3. Utilisation d'activateurs de PPAR-α,β selon l'une des revendications précédentes, dans laquelle les activateurs de PPAR-α,β appartiennent au groupe constitué par l'acide pirinixinique, le clofibrate, le leukotriène B4, l'acide 8-S-hydroxyeicosatétraénylique, le bézafibrate, la ciglitazone et la prostaglandine A2 ou leurs sels physiologiquement acceptables et les mélanges des composés mentionnés ci-dessus.
